# EUROPÄISCHE PATENTANMELDUNG

(11) **EP 2 050 483 A1**
(43) Veröffentlichungstag der Anmeldung: **22.04.2009**
(21) Anmeldenummer: 08018077.1
(22) Anmeldetag: 15.10.2008
(51) Int. Cl.: A61N 2/02, H01F 5/00

(54) **Spulenanordnung zum Erzeugen therapeutischer elektromagnetischer Felder**

(30) Priorität: 16.10.2007 DE 102007049541; 30.04.2008 DE 102008021573
(71) Anmelder: Neue Magnetodyn GmbH, 80333 München (DE)
(72) Erfinder: Kraus, Werner, 80539 München (DE); Kraus, Stephanie, 80538 München (DE); Stephan, Heribert, 80689 München (DE)
(74) Vertreter: Schumacher & Willsau

(57) **Zusammenfassung**

Die Erfindung betrifft eine Spulenanordnung (36) zum Erzeugen lokalisierter elektromagnetischer Felder, mit einer einen Kreuzungspunkt (38) aufweisenden Spulenwicklung, die durch eine achtförmige Gestalt zwei Flächen definiert (40, 42), wobei die Flächen (40, 42) so zueinander ausgerichtet sind, dass die von einem durch einen Stromfluss in der Spulenanordnung (36) erzeugten, die Flächen durchdringenden Magnetfelder (44, 46) im Wesentlichen gleichgerichtet sind.

## Beschreibung

Die Erfindung betrifft eine Spulenanordnung zum Erzeugen therapeutischer elektromagnetischer Felder.

Die Magnetfeldbehandlung von ausgedehnten Wunden des Weichgewebes, der Muskulatur und der Haut kann häufig den Heilungsprozess deutlich beschleunigen. Insbesondere existieren Erkrankungen, die im Hinblick auf eine antibiotische Behandlung therapieresistent sind, wobei letztere beispielsweise durch MRSA (Methicillin-resistenter Staphylococcus aureus), und andere Keime wie Enterokokken oder Pseudomonaden verursacht werden können. Diese Therapieresistenz kann durch die Magnetfeldbehandlung überwunden werden.

Therapieerfolge sind beispielsweise bei der Behandlung von schlecht heilenden Knochenfrakturen sowie damit in Verbindung stehender meist bakteriell verursachter Sekundärerkrankungen zu verzeichnen. Auch das so genannte "offene Bein" (Ulcus cruris) lässt sich mit Magnetfeldern erfolgreich therapieren. Weiterhin spielen dermatologische Anwendungen, beispielsweise zur Behandlung von großflächigen chronischen Wunden der Haut, wie Ulcera cruris oder Verbrennungen, im Zusammenhang mit der Magnetfeldtherapie eine wichtige Rolle. Zum Beispiel können Verbrennungen höheren Grades Hauttransplantationen erforderlich machen, die durch von außen angewendete Magnetfelder vorbereitet und nach der Transplantation beim Einheilen unterstützt werden können.

Die applizierten elektromagnetischen Wechselfelder haben beispielsweise eine Magnetfeldstärke von 1 bis 5 mT und einen sinusförmigen Verlauf mit einer Frequenz von 1 bis 20 Hz. Die Erzeugung der Felder erfolgt durch Magnetspulen, die mit einem entsprechenden Wechselstrom erregt werden. Dabei können verschiedene Spulenanordnungen zum Einsatzkommen, beispielsweise Solenoidspulen oder Helmholtzspulen, je nach Anwendung und gewünschter Magnetfeldrichtung.

Im Zusammenhang mit den verwendeten Spulen besteht der Wunsch, eine möglichst gut handhabbare Anordnung zur Verfügung zu stellen, die insbesondere transportabel und im Zusammenhang mit einer Vielzahl von Erkrankungen einsetzbar sein soll.

Der Erfindung liegt daher die Aufgabe zugrunde, eine Spulenanordnung zu schaffen, die in der Praxis bequem und flexibel einsetzbar ist.

Diese Aufgabe wird mit den Merkmalen des unabhängigen Anspruchs gelöst.

Vorteilhafte Ausführungsformen der Erfindung sind in den abhängigen Ansprüchen angegeben.

Die Erfindung besteht in einer Spulenanordnung zum Erzeugen lokalisierter elektromagnetischer Felder, mit einer einen Kreuzungspunkt aufweisenden Spulenwicklung, die durch eine achtförmige Gestalt zwei Flächen definiert, wobei die Flächen so zueinander ausgerichtet sind, dass die von einem durch einen Stromfluss in der Spulenanordnung erzeugten, die Flächen durchdringenden Magnetfelder im Wesentlichen gleichgerichtet sind. Die Wirkung von zwei getrennten Induktionsspulen mit gleicher Richtung ihres Magnetfeldes, in dem die lädierte Körperregion gelagert wird, kann auf diese Weise auch mit einer einzigen Spule erreicht werden. Dies macht die Anwendung komfortabel, insbesondere aufgrund des verminderten apparativen Aufwands.

Nützlicherweise ist vorgesehen, dass die Spulenanordnung flexibel ist, so dass die Flächen auf gegenüberliegenden Seiten einer einem Magnetfeld auszusetzenden Körperregion angeordnet werden können. Durch ihre flexible Wicklung, die insbesondere durch eine Elastizität der beteiligten Materialien bewirkt sein kann, wird die Umformung im Sinne eines Achter- oder Unendlich-Zeichens ermöglicht. Die sich durch diese Umformung der Spulenwicklung ergebenden "Schlaufen" der Spule können beispielsweise an beiden Seiten einer Extremität angelegt werden. Sie können je nach Anwendung gleiche oder unterschiedliche Größen haben. Die Spulenanordnung kann durch Ihre Flexibilität sehr vielseitig eingesetzt werden, insbesondere bei allen einleitend erwähnten Erkrankungen. Auch im Kieferbereich lässt sich die Spulenanordnung anwenden, indem die Schlaufen, ähnlich wie eine herabgezogene Schwimmbrille, auf beiden Seiten des Ober- und/oder Unterkiefers angeordnet werden. Metallische Kieferimplantate können dann in nützlicher Weise mit dem Magnetfeld wechselwirken, insbesondere auch Übertragerspulen oder Sekundärspulen, in welchen durch die als Primärspule wirkende erfindungsgemäße Spulenanordnung eine elektrische Spannung induziert wird. Diese elektrische Spannung kann bei geeigneter Kontaktierung gegeneinander elektrisch isolierte Dentalimplantatbereiche als Gewebeelektroden zur Wirkung bringen, so dass zusätzlich zur direkten Wirkung des Magnetfeldes auch therapeutisch nützliche elektrische Felder zur Verfügung stehen. Mit dieser Technik der induktiven Übertragung therapeutisch wirksamer Elektropotentiale auf die Dentalimplantate wird die Infektionsgefahr durch ansonsten bereits verwendete percutane Stromleitungen vermieden, und es können die Behandlungsparameter elektrische Spannung, Frequenz, Intensität, Signalform und die Behandlungszeit mit der indikationsspezifischen Programmierung eines Funktionsstrom-Generators des induzierenden Magnetfeldes bestimmt werden.

Im Zusammenhang mit der Auslegung der Spulenanordnung kann vorgesehen sein, dass an zwei dem Kreuzungspunkt abgewandten Positionen der Spulenanordnung zusammenwirkende Befestigungsmittel vorgesehen sind, durch die die Ausrichtung der Flächen zueinander geschaffen und aufrechterhalten werden kann. Die Befestigungsmittel können beispielsweise mittels Gurten, Druckknöpfen, Klettverschlüssen, Schnallen und dergleichen realisiert sein.

In besonders vorteilhafter Weise kann vorgesehen sein, dass der Kreuzungspunkt der Spulenanordnung durch eine Kopplungseinrichtung fixierbar ist. Eine solche Kopplungseinrichtung kann beispielsweise durch ein elastisches oder flexibles Band mit Klettverschluss oder Gürtelschließe realisiert sein.

Es ist von besonderem Vorteil, dass die Lage des Kreuzungspunktes und somit die Maße der Flächen variabel sind. Durch das auf diese Weise wählbare Flächenverhältnis der Spulenschlaufen ist die magnetische Induktionsflussdichte, die als Quotient aus dem magnetischen Fluss und der betrachteten Fläche definiert ist, einstellbar. Das Verhältnis der Induktionsflussdichten ist der Kehrwert des Verhältnisses der jeweils zugehörigen Flächen. Das Flächenverhältnis der beiden Schlaufen kann nach den therapeutischen Erfordernissen gewählt werden, indem eine veränderbare Fixierung des Kreuzungspunktes durch variable Anordnung der Kopplungseinrichtung ermöglicht wird. Beispielsweise kann in einem Zielbereich des Körpers eine hohe magnetische Induktionsflussdichte dadurch erzielt werden, dass eine kleinflächige Spulenschlaufe in dessen Nähe gebracht wird, während im Hinblick auf die andere großflächige Spulenfläche zur Bereitstellung des Helmholtzspuleneffektes hauptsächlich auf deren parallele Anordnung zur kleineren Fläche zu achten ist.

Gemäß einer besonders bevorzugten Ausführungsform der Erfindung ist die Spulenanordnung derart weitergebildet, dass sie plastische Eigenschaften hat. Durch die Flexibilität der Spule wird eine Anformung an die zu therapierende Körperregion ermöglicht, während die Plastizität die Spulenanordnung in der angeformten Position hält. Die plastischen Eigenschaften können somit unterstützend zu sonstigen Befestigungsvorrichtungen, wie etwa Schnallen oder Klemmen, wirken oder auch allein für den Halt der Spule in der gewünschten Form sorgen.

Nützlicherweise ist vorgesehen, dass die plastischen Eigenschaften durch mindestens eine plastische Materialkomponente vermittelt werden, die mindestens eine nicht plastische Materialkomponente umgibt und/oder von dieser eingebettet ist. Die Spule kann also einerseits aus einem flexiblen Material gefertigt werden, ohne dass es darauf ankäme, ob dieses plastische Eigenschaften hat. Die plastischen Eigenschaften werden der Spule dann durch eine plastische Materialkomponente vermittelt, so dass die Spule insgesamt in der durch die Verformung geschaffenen Stellung verbleibt.

Dies kann insbesondere dadurch realisiert sein, dass die mindestens eine plastische Materialkomponente durch mindestens eine sich im Wesentlichen parallel zu den Spulenwicklungen erstreckende Ader aus plastischem Material gebildet ist. Eine oder mehrere solche Adern verlaufen ganz oder teilweise parallel zu den Wicklungen der Spule. Eine in dieser Art und Weise aufgebaute Spule ist besonders einfach zu fertigen.

Nützlicherweise ist vorgesehen, dass die Spulenanordnung eine Spulenwicklung mit n Kreuzungspunkten aufweist, die n+1 Flächen definiert, wobei benachbarte Flächen aufgrund einer Flexibilität der Spulenanordnung verschiedene Winkel einschließen können, wobei bei einem eingeschlossenen Winkel von 180 Grad die benachbarte Flächen durchflutenden Magnetfelder entgegengesetzte Richtungen aufweisen und bei einem eingeschlossenen Winkel von 0 Grad die benachbarte Flächen durchflutenden Magnetfelder gleiche Richtungen aufweisen. Die im Zusammenhang mit dem erfindungsgemäßen System eingesetzte Spulenanordnung ist also nicht darauf beschränkt, in achtförmiger Gestalt eingesetzt zu werden, bei der ein Kreuzungspunkt und zwei Flächen vorliegen; vielmehr können auch kompliziertere geometrische Strukturen realisiert werden, zum Beispiel eine in der Weise geformte flexible Spule, die zwei Kreuzungspunkte und drei magnetisch relevante Flächen definiert. Sind die Kreuzungspunkte so gestaltet, dass diese durch eine Verwringung der Spulenfläche um 180° entstehen, so haben die durch die verschiedenen Flächen hindurchtretenden Magnetfelder jeweils umgekehrte Orientierung, wobei dies dann gilt, wenn außer der Verbindung der flexiblen Spule keine anderweitige Verformung vorgenommen wird. Die Spule kann aber in flexibler Weise anders geformt werden, so dass verschiedene Flächen jeweils paarweise so gekoppelt werden können, dass die hindurchtretenden Magnetfelder gleichgerichtet sind. Verwendet man beispielsweise eine Spule mit zwei Kreuzungspunkten und demnach drei Flächen, so kann die mittlere Fläche beispielsweise unter das Becken oder auf den Bauch oder auch um die Hüfte eines Patienten gelegt werden, während die seitlichen Flächen so geklappt werden, dass sie mit der mittleren Fläche einen Winkel von circa 180° bzw.90° einschließen und hierdurch beispielsweise nah an die Beckenknochen eines Patienten gebracht werden. Die magnetischen Feldlinien die dann durch eine der seitlichen Flächen hindurchtreten, verlaufen somit ebenfalls durch die mittlere Fläche.

Es ist von besonderem Vorteil, dass ein Winkel (α) sich kreuzender Spulenabschnitte einstellbar ist. Die magnetische Flussdichte in der Umgebung des Kreuzungspunktes der Spulenwicklung ist abhängig von der Richtung der Spulenströme, die die Wicklungsabschnitte der Kreuzung durchfließen. Die magnetische Flussdichte kann von ihrem bei einem stumpfen Kreuzungswinkel vorliegenden Wert bis zum nahezu doppelten Wert bei spitzen Winkeln gesteigert werden, was durch Übergang zu annähernd parallelem Stromverlauf in den Wicklungsabschnitten der Kreuzung erreicht werden kann. Diese Möglichkeit der lokalen Steigerung der Flussdichte im Bereich der Wicklungskreuzung ist von erheblicher Bedeutung für beispielsweise die Therapie schlecht heilender Operationswunden der Haut und des Unterhautgewebes nach Osteosynthesen bei Risikopatienten, das heißt insbesondere Durchblutungsgestörte Patienten, Diabetiker, Raucher und Patienten mit kritischen Cortisonwerten und andere.

Die Einstellbarkeit des Winkels kann dadurch realisiert sein, dass sich kreuzende Spulenabschnitte durch eine Kopplungseinrichtung mit mindestens zwei relativ zueinander beweglichen Komponenten geführt sind, wobei der Winkel der sich kreuzenden Spulenabschnitte durch eine Relativbewegung der Komponenten einstellbar ist. Die beiden beweglichen Komponenten können beispielsweise als zwei Kreisscheiben mit jeweiliger Führung für die sich kreuzenden Spulenabschnitte realisiert sein. Indem die Kreisscheiben um ihre gemeinsame Achse relativ zueinander gedreht werden, ändert sich auch der Winkel zwischen den Spulenabschnitten und somit die lokale magnetische Flussdichte.

Mit der erfindungsgemäßen Spulenanordnung wird erreicht, dass das Magnetfeld der beiden sich gegenüberstehenden Schlaufen der Spulenanordnung durch die räumliche Umkehr der Stromrichtung in einer der beiden Schlaufen, wie bei der Anordnung von zwei getrennten Spulen nach Helmholtz, gleichgerichtet ist, wobei eine besonders gute und flexible Handhabbarkeit zur Verfügung gestellt wird. Eine einfache Handhabung bei der Anpassung der geometrischen Form der Spulenanordnung an die Lage der jeweiligen Knochen bzw. Weichgewebeläsion wird ermöglicht. Beispielsweise können die Schlaufenflächen an Behandlungsbereiche wie Fuß, Knie Unterschenkel, Oberschenkel, Becken, Wirbelsäule, Hand, Unterarm, Oberarm, Kiefer und Schädelbereich angepasst werden; durch Variation der Schlaufengrößen auch im Hinblick auf die Stärke des Magnetfeldes. Eine weitere Eigenschaft der erfindungsgemäßen Spulenanordnung ist eine Zunahme der Flussdichte im Nahbereich des Kreuzungspunktes, so dass eine relativ starke Magnetfeldkonzentration auf eine kleine Körperfläche ermöglicht wird. Auf diese Weise lassen sich stark lokalisierte Erkrankungen, wie zum Beispiel Abszesse und Infekte, wirkungsvoll behandeln.

Die Erfindung wird nun mit Bezug auf die begleitenden Zeichnungen anhand besonders bevorzugter Ausführungsformen beispielhaft erläutert.

Es zeigen:
- Figur 1: eine flexible Spulenanordnung;
- Figur 2: eine erfindungsgemäße flexible Spulenanordnung in einem Zustand außerhalb ihrer Anwendung;
- Figur 3: eine perspektivische Darstellung einer erfin- dungsgemäßen Spulenanordnung während ihrer Anwen- dung;
- Figur 4: eine schematische Darstellung zur Erläuterung der räumlichen Ausrichtung eines durch eine erfin- dungsgemäße Spulenanordnung erzeugten Magnetfel- des;
- Figur 5: eine geschnittene perspektivische Teildarstellung einer Spulenanordnung mit plastischen Eigenschaf- ten;
- Figur 6: eine erfindungsgemäße flexible Spulenanordnung in einem weiteren Zustand außerhalb ihrer Anwendung;
- Figur 7: eine erfindungsgemäße flexible Spulenanordnung in einem weiteren Zustand außerhalb ihrer Anwendung;
- Figur 8: Komponenten einer Kopplungseinrichtung zur Ein- stellung einer erfindungsgemäßen Spulenanordnung und
- Figur 9: eine Kopplungseinrichtung zur Einstellung einer erfindungsgemäßen Spulenanordnung.

Bei der nachfolgenden Beschreibung der Zeichnungen bezeichnen gleiche Bezugszeichen gleiche oder vergleichbare Komponenten.

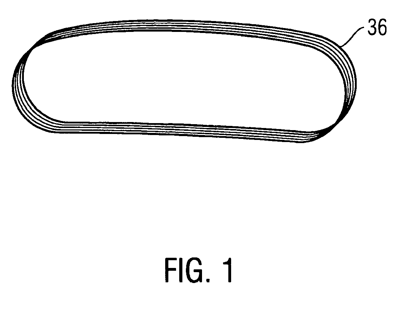

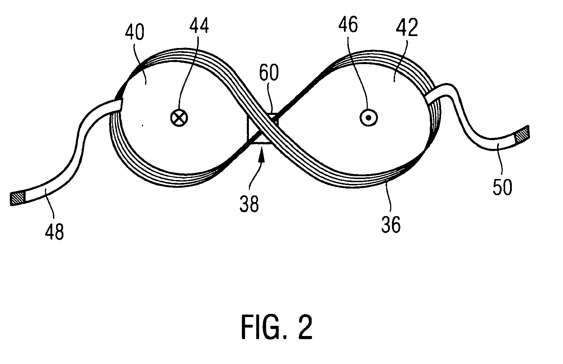

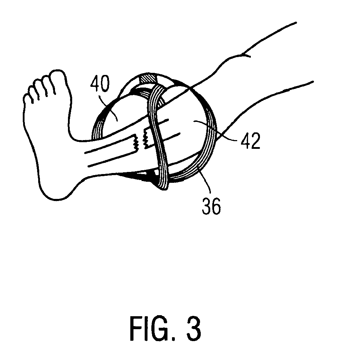

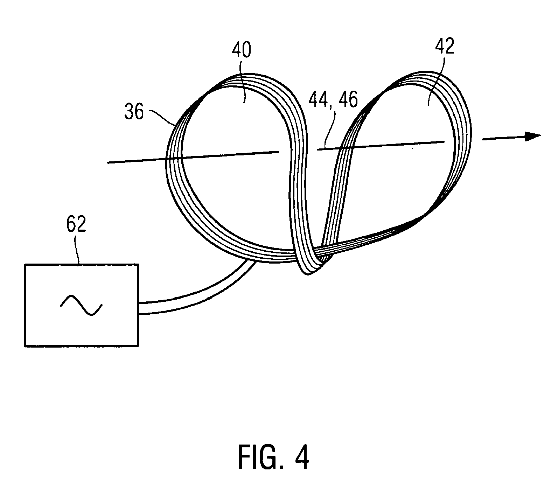

Figur 1 zeigt eine flexible Spulenanordnung. Figur 2 zeigt eine erfindungsgemäße flexible Spulenanordnung in einem Zustand außerhalb ihrer Anwendung. Figur 3 zeigt eine perspektivische Darstellung einer erfindungsgemäßen Spulenanordnung während ihrer Anwendung. Figur 4 zeigt eine schematische Darstellung zur Erläuterung der räumlichen Ausrichtung eines durch eine erfindungsgemäße Spulenanordnung erzeugten Magnetfeldes. Nutzt man die Flexibilität der Spulenanordnung 36 dazu aus, diese in eine achtförmige Gestalt zu bringen, so werden zwei Flächen 40, 42 sowie ein Kreuzungspunkt 38 definiert. Der Kreuzungspunkt 38 ist durch eine Kopplungseinrichtung 60 fixiert und kann vorzugsweise verschoben werden, so dass das Verhältnis der Flächen 40, 42 variabel ist. Beispielsweise kann als Kopplungseinrichtung 60 ein elastisches Band mit Klettverschluss oder Gürtelschließe dienen. Ebenfalls ist es denkbar, Oberflächen der Spulenanordnung so auszustatten, dass sie die Komponenten eines Klettverschlusses bilden und auf diese Weise an verschiedenen Stellen direkt aufeinander Haften können. Um die Einstellung zu erleichtern, können im Variationsbereich Vermerke oder Skalen vorgesehen sein; an diesen kann sich der Anwender orientieren, wenn er eine bestimmte Fixierung des Kreuzungspunktes 38 vornehmen möchte. Fließt durch eine solche Spulenanordnung 36 ein Wechselstrom, so haben die induzierten Magnetfelder entgegengesetzte Richtungen, wie durch die Vektorsymbole 44, 46 angedeutet ist. Aufgrund der Flexibilität der Spulenanordnung 36 kann diese aber auch in eine andere Gestalt gebracht werden. Dies ist im Zusammenhang mit Figur 3 dargestellt, wo auch ein Bein mit einem frakturierten Knochen als Beispiel einer Körperregion mit irgendeiner der vorstehend erwähnten Krankheiten gezeigt ist. Nun liegen sich die Flächen 40 ,42 gegenüber und die in den jeweiligen Segmenten der Spule erzeugten Magnetfelder haben die gleiche Richtung. Dies ist in Figur 4, in der auch ein mit der Spulenanordnung gekoppelter Wechselstromgenerator 62 gezeigt ist, nochmals veranschaulicht, nämlich durch den einheitlichen Magnetfeldvektor 44, 46, der beide Flächen 40, 42 durchdringt. Gemäß Figur 3 durchdringt das Magnetfeld den erkrankten Körperbereich im Wesentlichen senkrecht zur Längsachse der Extremität. Ebenfalls ist es möglich, dass die Extremität die von den Schlaufen der achtförmigen Spule definierten Flächen durchdringt, so dass dann das Magnetfeld im Wesentlichen parallel zur Achse der Extremität verläuft.

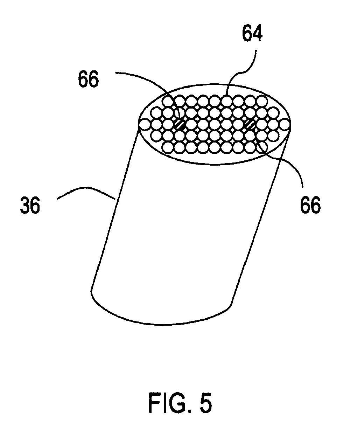

Figur 5 zeigt eine geschnittene perspektivische Teildarstellung einer Spulenanordnung mit plastischen Eigenschaften. Innerhalb der Spulenanordnung 36 ist die elektrisch leitende Spulenwicklung 64 zu erkennen. Zusätzlich sind parallel zu der Spulenwicklung 64 zwei Adern 66 aus plastischem Material vorgesehen, die der Spulenanordnung 36 insgesamt eine plastische Flexibilität vermitteln.

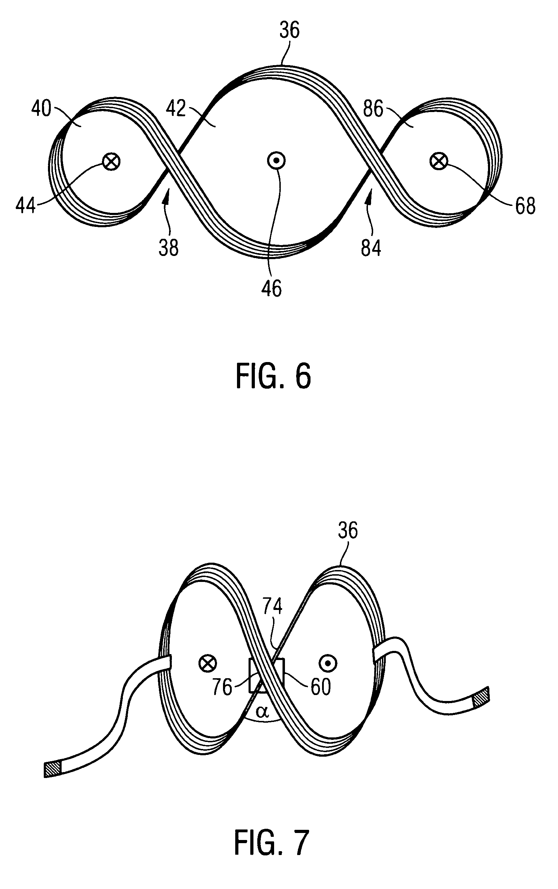

Figur 6 zeigt eine erfindungsgemäße elastische Spulenordnung in einem weiteren Zustand außerhalb ihrer Anwendung. Im Vergleich zu der Ausführungsform gemäß Figur 2 weist die hier dargestellte Spulenordnung nicht einen Kreuzungspunkt 38 und zwei magnetisch wirksame Flächen 40, 42, sondern zwei Kreuzungspunkte 38, 64 und drei magnetisch wirksame Flächen 40, 42, 66 auf. Die mittlere Fläche 42 ist größer als die äußeren Flächen 40, 66, wodurch die Spulenanordnung so eingesetzt werden kann, dass beispielsweise der mittlere Teil der Spulenanordnung, welcher die Fläche 42 definiert die unter dem Becken, oder auf dem Bauch zu Liegen kommt oder die Hüfte eines Patienten umgibt, während die äußeren Schlingen der Spulenanordnung, welche die kleineren Flächen 40, 66 definieren, nach Bedarf ausgerichtet werden, beispielsweise in dem Bereich der Beckenknochen geklappt werden und einen Winkel von 180° bzw. 90° mit der großen Schlinge bilden. Zahlreiche weitere Variationsmöglichkeiten sind denkbar. Beispielsweise ist es auch möglich, die äußeren Flächen 40, 66 in den Bereich der Oberschenkel zu bringen, um hier positiv auf Frakturen oder Nekrosen der Beckenknochen, der Hüftgelenke oder der Hüfte einzuwirken. Die Ausführungsform gemäß Figur 6 macht deutlich, dass die erfindungsgemäße Spulenanordnung aufgrund ihrer Flexibilität besonders variantenreich einsetzbar ist. Dies betrifft sowohl die Formgebung der Spulenanordnung 36 als auch die magnetischen Eigenschaften. In Figur 6 ist eine Verformung der Spulenanordnung 36 dargestellt, bei der die Magnetfelder 44, 46, 68 benachbarter Flächen 40, 42, 66 jeweils entgegengesetzte Richtungen aufweisen. Es ist aber auch denkbar, dass man beispielsweise die die Fläche 66 definierende Schlaufe unter nochmaliger Verwringung des Kreuzungspunktes 64 so anordnet, dass die die benachbarten Flächen 42, 66 durchdringenden Magnetfelder gleichgerichtet sind. Bewegt man dann beispielsweise die seitlichen Flächen 40, 66 so, dass sie Ebenen senkrecht zur Papierebene definieren, so können die diese Magnetfelder durchdringenden Feldlinien miteinander koppeln, während bei einer Auslegung der Magnetfelder, wie sie in Figur 6 dargestellt ist, immer eine Kopplung der die seitlichen Flächen durchdringenden Magnetfelder mit dem die zentrale Fläche 42 durchdringenden Magnetfeld zu erwarten ist.

Figur 7 zeigt eine erfindungsgemäße elastische Spulenanordnung in einem weiteren Zustand außerhalb ihrer Anwendung. Die hier dargestellte Ausführungsform ähnelt derjenigen gemäß Figur 2. Allerdings ist der Winkel α, mit dem sich die Spulenabschnitte 74, 76 kreuzen, hier spitzer als bei der Auslegung gemäß Figur 2. In dem Ausführungsbeispiel gemäß Figur 2 stehen die Stromvektoren der sich kreuzenden Spulenabschnitte nahezu senkrecht aufeinander, während bei der Ausführungsform gemäß Figur 7 im Kreuzungspunkt nicht senkrechte Stromkomponenten vorliegen und insofern parallele Vektorkomponenten des Stroms wirksam sind. Dies führt zu einer Erhöhung der magnetischen Flussdichte im Kreuzungspunkt der Spulenanordnung 36, so dass lokal eine Verstärkung der magnetischen Flussdichte erzielt werden kann. Die vorteilhafte Verstärkung der Flussdichte kann auch bei Kreuzungspunkten komplexerer Spulenformen realisiert werden, beispielsweise bei derjenigen gemäß Figur 6.

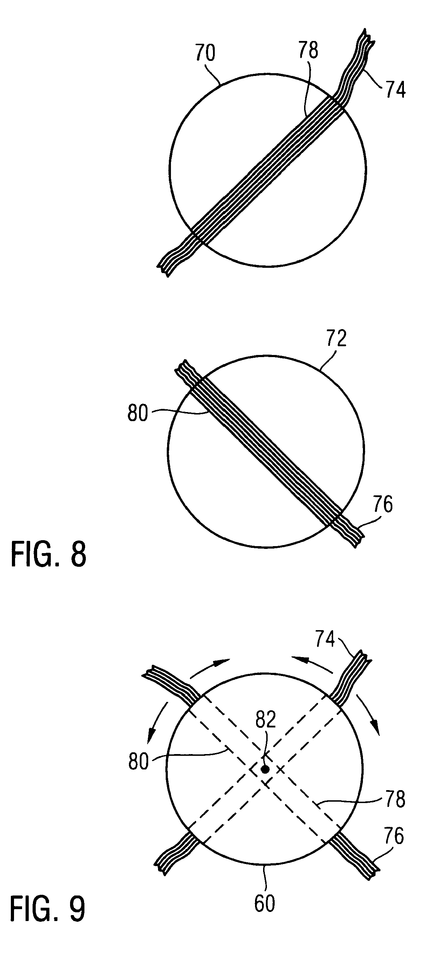

Figur 8 zeigt Komponenten einer Kopplungseinrichtung zur Einstellung einer erfindungsgemäßen Spulenanordnung und Figur 9 zeigt eine Kopplungseinrichtung zur Einstellung einer erfindungsgemäßen Spulenanordnung. Es ist veranschaulicht in welcher Weise die Variabilität des Winkels von sich kreuzenden Spulenabschnitten 74, 76 erreicht werden kann. Im vorliegenden Beispiel wird dies durch zwei Komponenten 70, 72 geleistet, die je einen Spulenabschnitt 74, 76 in jeweils einer länglichen Ausnehmung 78, 80 führen. Sind diese beiden Komponenten 70, 42 gegeneinander um ihre gemeinsame Achse 82 drehbar verbunden, so kann durch eine solche Drehung, die in Figur 9 durch die Pfeile angedeutet ist, eine Änderung des Winkels im Bereich des Kreuzungspunktes erreicht werden.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste:

- 36: Spulenanordnung
- 38: Kreuzungspunkt
- 40: Fläche
- 42: Fläche
- 44: Magnetfeld / Magnetfeldvektor / Vektorsymbol
- 46: Magnetfeld / Magnetfeldvektor / Vektorsymbol
- 48: Befestigungsmittel
- 50: Befestigungsmittel
- 60: Kopplungseinrichtung
- 62: Wechselstromgenerator
- 64: Spulenwicklung
- 66: Plastische Ader
- 68: Magnetfeld / Magnetfeldvektor / Vektorsymbol
- 70: Komponente der Kopplungseinrichtung
- 72: Komponente der Kopplungseinrichtung
- 74: Spulenabschnitt
- 76: Spulenabschnitt
- 78: Ausnehmung
- 80: Ausnehmung
- 82: Achse
- 84: Kreuzungspunkt
- 86: Fläche

## Patentansprüche

1. Spulenanordnung zum Erzeugen lokalisierter elektromagnetischer Felder, mit einer einen Kreuzungspunkt (38) aufweisenden Spulenwicklung, die durch eine achtförmige Gestalt zwei Flächen definiert (40, 42), wobei die Flächen (40, 42) so zueinander ausgerichtet sind, dass die von einem durch einen Stromfluss in der Spulenanordnung (36) erzeugten, die Flächen durchdringenden Magnetfelder (44, 46) im Wesentlichen gleichgerichtet sind.

2. Spulenanordnung nach Anspruch 1, **dadurch gekennzeichnet, dass** die Spulenanordnung (36) flexibel ist, so dass die Flächen (40, 42) auf gegenüberliegenden Seiten einer einem Magnetfeld auszusetzenden Körperregion angeordnet werden können.

3. Spulenanordnung nach Anspruch 1 oder 2, **dadurch gekennzeichnet, dass** an zwei dem Kreuzungspunkt (38) abgewandten Positionen der Spulenanordnung zusammenwirkende Befestigungsmittel (48, 50) vorgesehen sind, durch die die Ausrichtung der Flächen (40, 42) zueinander geschaffen und aufrechterhalten werden kann.

4. Spulenanordnung nach einem der vorhergehenden Ansprüche, **dadurch gekennzeichnet, dass** der Kreuzungspunkt (38) der Spulenanordnung durch eine Kopplungseinrichtung (60) fixierbar ist.

5. Spulenanordnung nach einem der vorhergehenden Ansprüche, **dadurch gekennzeichnet, dass** die Lage des Kreuzungspunktes (38) und somit die Maße der Flächen (40, 42) variabel sind.

6. Spulenanordnung nach einem der vorhergehenden Ansprüche, **dadurch gekennzeichnet, dass** sie plastische Eigenschaften hat.

7. Spulenanordnung nach Anspruch 6, **dadurch gekennzeichnet, dass** die plastischen Eigenschaften durch mindestens eine plastische Materialkomponente vermittelt werden, die mindestens eine nicht plastische Materialkomponente umgibt und/oder von dieser eingebettet ist.

8. Spulenanordnung nach einem der vorhergehenden Ansprüche, **dadurch gekennzeichnet, dass** die mindestens eine plastische Materialkomponente durch mindestens eine sich im Wesentlichen parallel zu den Spulenwicklungen erstreckende Ader aus plastischem Material gebildet ist.

9. Spulenanordnung nach einem der vorhergehenden Ansprüche, **dadurch gekennzeichnet, dass** die Spulenanordnung (36) eine Spulenwicklung mit n Kreuzungspunkten (38, 84) aufweist, die n+1 Flächen (40, 42, 86) definiert, wobei benachbarte Flächen aufgrund einer Flexibilität der Spulenanordnung verschiedene Winkel einschließen können, wobei bei einem eingeschlossenen Winkel von 180 Grad die benachbarte Flächen durchflutenden Magnetfelder (44, 46, 68) entgegengesetzte Richtungen aufweisen und bei einem eingeschlossenen Winkel von 0 Grad die benachbarte Flächen durchflutenden Magnetfelder gleiche Richtungen aufweisen.

10. Spulenanordnung nach einem der vorhergehenden Ansprüche, **dadurch gekennzeichnet, dass** ein Winkel (α) sich kreuzender Spulenabschnitte (74, 76) einstellbar ist.

11. Spulenanordnung nach Anspruch 10, **dadurch gekennzeichnet, dass** sich kreuzende Spulenabschnitte (74, 76) durch eine Kopplungseinrichtung (60) mit mindestens zwei relativ zueinander beweglichen Komponenten (70, 72) geführt sind, wobei der Winkel (α) der sich kreuzenden Spulenabschnitte (74, 76) durch eine Relativbewegung der Komponenten (70, 72) einstellbar ist.
